# EUROPÄISCHE PATENTANMELDUNG

(11) **EP 2 500 043 A1**
(43) Veröffentlichungstag der Anmeldung: **19.09.2012**
(21) Anmeldenummer: 12157486.7
(22) Anmeldetag: 29.02.2012
(51) Int. Cl.: A61L 29/08, A61L 29/16, A61L 31/02, A61L 31/10, A61L 31/16

(54) **Medizinprodukt mit einer aktiven Beschichtung**

(30) Priorität: 16.03.2011 US 201161453146 P
(71) Anmelder: Biotronik AG, 8180 Bülach (CH)
(72) Erfinder: Wittchow, Eric, 90403 Nürnberg (DE); Groschner, Klaus, 8075 Hart bei Graz (AT); Braune, Marlen, 91080 Marloffstein (DE)
(74) Vertreter: Lindner-Vogt, Karin L.

(57) **Zusammenfassung**

Die vorliegende Erfindung betrifft ein mit einer Polymerschicht überzogenes Medizinprodukt, welches unter, in und/oder auf der biostabilen und/oder bioabbaubaren Polymerschicht einen Inhibitor des TRPC-3 Ionenkanals aufweist.

## Beschreibung

Die vorliegende Erfindung betrifft ein mit einer Polymerschicht überzogenes Medizinprodukt, welches unter, in und/oder auf der biostabilen und/oder bioabbaubaren Polymerschicht einen innerhalb der TRP-Familie selektiven Inhibitor des TRPC-3 Ionenkanals aufweist.

Medizinische Endoprothesen oder Medizinprodukte bzw. Implantate für die unterschiedlichsten Anwendungen sind in großer Vielfalt aus dem Stand der Technik bekannt. Sie dienen beispielsweise der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, z.B. als Nagel, Platte oder Schraube.

In einer heutzutage häufig angewendeten Therapie, insbesondere bei Herz-Kreislauf-Erkrankungen, werden Stents eingesetzt. Diese dienen dazu, Hohlorgane durchgängig zu halten. Sie weisen häufig einen Körper in der Form eines ggf. durchbrochenen rohrförmigen oder hohlzylinderförmigen Grundgitters auf, das an beiden Längsenden offen ist. Das rohrförmige Grundgitter einer derartigen Endoprothese wird in das zu behandelnde Hohlorgan eingesetzt und stützt dort das behandelte Hohlorgan. Stents haben sich insbesondere zur Behandlung von Blutgefäßerkrankungen etabliert. Durch den Einsatz von Stents, ggf. unter Zuhilfenahme z.B. eines Ballonkatheters, können verengte Bereiche in Blutgefäßen erweitert und offen gehalten werden, so dass ein Lumengewinn resultiert. Stents können aber auch beispielsweise bei der Krebsbehandlung dazu dienen, durch bösartige Tumore verursachte Verengungen von Atemwegen (z.B. der Luftröhre), Gallenwegen oder der Speiseröhre nach einer Aufdehnung offen zu halten.

Durch den Einsatz von Stents oder anderen Medizinprodukten kann zwar ein für den Therapieerfolg primär notwendiger optimaler Querschnitt des Hohlorgans erreicht werden, allerdings initiiert die dauerhafte Anwesenheit eines derartigen Fremdkörpers eine Kaskade von biologischen Prozessen, die beispielsweise eine Entzündung (Inflammation) des behandelten Hohlorgans oder eine nekrotische Veränderung begünstigen und die zu einem allmählichen Zuwachsen des gestenteten Blutgefäßes führen können. Im schlimmsten Fall kann diese Veränderung des Hohlorgans eine erneute Verengung (Restenose) des Hohlorgans - bis hin zum Verschluss - bedingen.

Es ist wünschenswert, dass die oben beschriebene, eine Inflammation begünstigende Wirkung von Medizinprodukten in Zukunft weitestgehend vermieden wird, da hierdurch die Wirksamkeit des Medizinprodukts verringert wird und weitere Schädigungen des behandelten Organismus hervorgerufen werden können.

Seit einigen Jahren versucht man die Restenosegefahr bei der Implantation von Stents durch Aufbringung spezieller Beschichtungen zu mindern. Teilweise dienen die Beschichtungssysteme selbst als Trägermatrix, in die ein oder mehrere Arzneistoffe eingebettet sind (Local Drug Delivery). Die Beschichtung bedeckt in der Regel zumindest eine der Gefäßwand zugewandte Oberfläche des endovaskulären Implantats.

Die Beschichtungen bestehen nahezu zwangsläufig aus einem biokompatiblen Material, welches entweder natürlichen Ursprungs ist oder auf synthetischem Wege gewonnen werden kann. Eine besonders gute Verträglichkeit und die Möglichkeit, die Elutionscharakteristik des eingebetteten Arzneistoffs zu beeinflussen, bieten biodegradierbare Beschichtungsmaterialien. Beispiele für die Verwendung biodegradierbarer Polymere sind Cellulose, Kollagen, Albumin, Casein, Polysaccharide (PSAC), Polylactid (PLA), Poly-L-lactid (PLLA), Polyglykol (PGA), Poly-D-L-lactid-CO-glycolid( PDLLA/PGA), Polyhydroxybuttersäure (PHB), Polyhydroxyvaleriansäure (PHV), Polyalkylcarbonate, Polyorthoester, Polyethylenterephthalat (PET), Polymalonsäure (PML), Polyanhydride, Polyphosphazene, Polyaminosäuren und deren Copolymere sowie Hyaluronsäure und seine Derivate. Zahlreiche Studien belegen mittlerweile den positiven Effekt von biokompatiblen Beschichtungen auf die Restenoseneigung bei metallischen Stents. Trotz dieser Erfolge verbleibt auch bei den bisher eingesetzten Materialien noch ein nicht unerhebliches Restrisiko zur Restenosebildung.

Viel versprechende Ansätze zur Verringerung des Restenoserisikos stellen beschichtete wirkstoffeluierende Stents dar, die bedingt durch den Wirkstoff, Einfluss auf die wichtigsten Zellfunktionen nehmen.

Dies gelingt unter anderem durch die Aktivierung oder Inhibierung von in der Zellmembran vorliegenden Ionenkanälen, welche die unterschiedlichen Zellfunktionen steuern. Eine der umfangreichsten Familien der zellulären Ionenkanäle stellen die TRP Ionenkanäle (Transient Receptor Potential) mit der Unterfamilie TRPC Ionenkanäle (Transient Receptor Potential Classical oder Canonical) dar.

Fast alle Mitglieder der TRPC-Familie werden auch in den glatten Muskelzellen der Gefäßmuskulatur exprimiert. Die glatten Muskelzellen stellen einen maßgeblichen Faktor bei einer Restenose dar. Bedingt durch eine Gewebsreizung bei der Implantation wird in diesen Zellen ein Reparaturmechanismus ausgelöst, der eine erhöhte Proliferation der Zellen zur Folge hat. Allerdings ist bislang unbekannt, dass durch eine gezielte Blockade spezieller mit Proliferation assoziierter Ionenkanäle wie dem TRPC-3 der komplexe Prozess der Restenose vorteilhaft beeinflusst werden kann.

Der Erfindung liegt die Aufgabe zu Grunde, ein Medizinprodukt bereitzustellen, das durch die Verwendung eines hochaffinen und selektiven Inhibitors des TRPC-3 Ionenkanals eine erhöhte Zellproliferation an glatten Muskelzellen verringert oder vermeidet.

Diese Aufgabe wird erfindungsgemäß dadurch gelöst, dass ein ganz oder in Teilen mit einer biostabilen und/oder bioabbaubaren Polymerschicht überzogenes Medizinprodukt bereitgestellt wird, welches unter, in und/oder auf der biostabilen und/oder bioabbaubaren Polymerschicht einen Inhibitor des TRPC-3 Ionenkanals aufweist.

Durch die lokale Anwendung selektiver Inhibitoren mit einer hohen Affinität gegenüber den zellulären TRPC-3 Ionenkanälen konnte erstmalig gezeigt werden, dass die Proliferation von glatten Muskelzellen nach einer Stentimplantation deutlich verringert werden konnte. Dieses Ergebnis ist umso überraschender, da andere Calciumkanalblocker, die nicht selektiv auf einen der TRPC-Kanäle wirken, sondern einem anderen Wirkmechanismus folgen, im klinischen Einsatz keine antirestenotische Wirkung aufweisen. (Jørgensen B et al, Restenosis and Clinical Outcome in Patients Treated With Amlodipine After Angioplasty: Results From the Coronary AngioPlasty Amlodipine REStenosis Study (CAPARES). J Am Coll Cardiol 2000;35:592-9)

Im Folgenden steht der Begriff Inhibitor als Äquivalent für Inhibitor des TRPC-3 Ionenkanals.

Unter einem Inhibitor im Rahmen der vorliegenden Erfindung ist ein Hemmstoff (also eine Substanz) zu verstehen, der eine oder mehrere Reaktionen - chemischer, biologischer oder physiologischer Natur - so beeinflusst, dass diese verlangsamt, gehemmt oder verhindert werden. Beispielsweise stellt ein Merkmal einer TRPC-3 Inhibierung die Verhinderung von Calcium-Einstrom in die Zelle dar. Bei dem erfindungsgemäßen Inhibitor handelt es sich um einen antiproliferativen Wirkstoff, d.h. einen Wirkstoff, der so wirkt, dass er vaskuläres Wachstum durch Unterdrückung der dehnungsinduzierten NF-AT-Aktivierung inhibiert.

In einer bevorzugten Ausführung der vorliegenden Erfindung ist der Inhibitor ein Amidophenylpyrazol oder ein Derivat hiervon, insbesondere ein Amidophenyl-5-trifluormethylpyrazol oder ein Derivat hiervon. Insbesondere bevorzugt ist der Inhibitor Ethyl-1-(4-(2,3,3-trichloracrylamid)phenyl)-5-(trifluormethyl)-1H-pyrazol-4-carboxylat (Pyr3).

Unter einem Derivat des Amidophenyl-5-trifluormethylpyrazols versteht man im Rahmen der vorliegenden Erfindung ein Amidophenylpyrazol, das zusätzlich am Pyrazol-Ring und/oder an der Amid-Funktion einen oder mehrere verschiedene oder gleiche Substituenten trägt. Zu den Substituenten zählen beispielsweise, aber nicht einschränkend, Trifluormethyl-, Phenyl- und einfach oder mehrfach halogenierte Derivate hiervon, Trichlorethylen, Pyridinyl und halogenierte Derivate hiervon, sowie 5- oder 6-gliedrige Heterocyclen, die einen oder mehrere Heteroatome wie N, S oder O enthalten können. Vorteilhafterweise ist der oder sind die Substituenten so ausgewählt, dass die Lipophilie des Amidopyrazols erhöht wird, um einerseits die Eluierung des oder der Wirkstoffe aus der polymeren Trägermatrix besser zu steuern und andererseits die Geweberetentionszeit zu erhöhen.

Es hat sich überraschenderweise gezeigt, dass durch das Ein- und/oder Aufbringen eines selektiven Inhibitors des TRPC-3 Ionenkanals, insbesondere Pyr3, unter, in und/oder auf der biostabilen und/oder bioabbaubaren Polymerschicht die Proliferation glatter Muskelzellen inhibiert werden kann. Aufgrund der hohen Affinität von Pyr3 an den TRPC-3 Ionenkanal ist es somit möglich, ein Medizinprodukt bereitzustellen, das nach der Implantation ein geringeres Restenoserisiko aufweist, da die Proliferation der glatten Muskelzellen effektiv inhibiert wird.

Medizinprodukte innerhalb des Schutzumfangs der vorliegenden Erfindungen beinhalten beliebige medizinische Vorrichtungen, welche benutzt werden, wenigstens teilweise, um in den Körper eines Patienten eingebracht zu werden. Beispiele beinhalten implantierbare Vorrichtungen, Herzschrittmacher, Katheter, Nadelinjektionskatheter, Blutgerinnselfilter, vaskuläre Transplantate, Ballons, Stenttransplantate, Gallenstents, Darmstents, Bronchiallungenstents, Speiseröhrenstents, Harnleiterstents, Aneurysmen-ausfüllende Spulen und andere Spulenvorrichtungen, transmyokardiale Revaskularisationsvorrichtungen, perkutane myokardiale Revaskularisationsvorrichtungen. Weiterhin können beliebige natürliche und/oder künstliche Medizinprodukte eingesetzt werden, beispielsweise Prothesen, Organe, Gefäße, Aorten, Herzklappen, Schläuche, Organersatzteile, Implantate, Fasern, Hohlfasern, Membranen, Konserven, Blutbehältern, Titerplatten, Adsorbermedien, Dialysatoren, Verbindungsstücke, Sensoren, Ventile, Endoskope, Filter, Pumpenkammern sowie andere Medizinprodukte, welche hämokompatible Eigenschaften aufweisen sollen. Der Begriff Medizinprodukte ist weit zu fassen und bezeichnet insbesondere solche Produkte, die kurzzeitig (z.B. Endoskope) oder dauerhaft (z.B. Stents) mit Blut in Kontakt kommen.

Besonders bevorzugte Medizinprodukte sind Ballonkatheter und endovaskuläre Prothesen, insbesondere Stents.

Stents herkömmlicher Bauart weisen eine filigrane Stützstruktur aus metallischen Streben auf, die zur Einbringung in den Körper zunächst in einem nicht-expandierten Zustand vorliegen und die am Ort der Applikation dann in einen expandierten Zustand aufgeweitet werden. Der Stent kann vor oder nach dem Crimpen auf einen Ballon beschichtet werden.

Der Grundkörper des Stents besteht vorzugsweise aus einem metallischen Material aus einem oder mehreren Metallen aus der Gruppe Eisen, Magnesium, Nickel, Wolfram, Titan, Zirkonium, Niob, Tantal, Zink, Platin, Iridium oder Silizium und ggf. einer zweiten Komponente aus einem oder mehreren Metallen aus der Gruppe Lithium, Natrium, Kalium, Kalzium, Mangan, Eisen oder Wolfram, vorzugsweise aus einer Zink-Kalziumlegierung.

In einem weiteren Ausführungsbeispiel besteht der Grundkörper aus einem Formgedächtnis-Material aus einem oder mehreren Materialien aus der Gruppe bestehend aus Nickel-Titan-Legierungen und Kupfer-Zink-Aluminium-Legierungen, vorzugsweise aber aus Nitinol.

In einem weiteren bevorzugten Ausführungsbeispiel besteht der Grundkörper des Stents aus Edelstahl, vorzugsweise aus einem Cr-Ni-Fe-Stahl - hier bevorzugt die Legierung 316L - oder einem Co-Cr-Stahl. Ferner kann der Grundkörper des Stents mindestens teilweise aus Kunststoff und/oder einer Keramik bestehen.

Zusätzlich kann auf dem Grundkörper des Stents, bestehend aus einem metallischen Material, eine passivierende Siliziumcarbid-Schicht (SiC) vorgesehen sein. Diese wird nach dem Fachmann bekannten Verfahren aufgebracht und befindet sich unter der Schicht enthaltend den Inhibitor des TRPC-3.

In einem weiteren Ausführungsbeispiel besteht der Grundkörper des Stents aus einem biokorrodierbaren metallischen Werkstoff, zum Beispiel einer biokorrodierbaren Legierung, ausgewählt aus der Gruppe Magnesium, Eisen und Wolfram; insbesondere ist der biokorrodierbare metallische Werkstoff eine Magnesiumlegierung.

Unter einer biokorrodierbaren Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seitenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

In einem weiteren Ausführungsbeispiel besteht der Stent aus natürlichen Polymeren, wie zum Beispiel aus Collagen, Chitin, Chitosan, Heparin. In einem weiteren Ausführungsbeispiel besteht der Stent aus abbaubaren Polymeren, wie zum Beispiel aus einem Polylactid wie PDLA, PLLA, PLGA, sowie P3HB, P4HB und Copolymeren aus den genannten Polymeren.

Das Stentdesign sollte vorzugsweise derart angepasst sein, dass ein möglichst großer Kontakt zur Gefäßwand besteht. Dies begünstigt eine gleichmäßige Elution des pharmakologischen Wirkstoffs.

Die Oberfläche des erfindungsgemäßen Medizinprodukts, bspw. Ballon oder Stent, dessen Grundkörper aus einem metallischen Material aus einem oder mehreren Metallen oder aus einem biokorrodierbaren metallischen Werkstoff oder aus einem Polymer besteht, weist ganz oder in Teilen eine biostabile und/oder bioabbaubare Polymerschicht auf, die einen Inhibitor des TRPC-3 Ionenkanals, bevorzugt ein Amidophenylpyrazol oder ein Derivat hiervon, insbesondere ein Amidophenyl-5-trifluormethylpyrazol oder ein Derivat hiervon, insbesondere bevorzugt Pyr3, enthält.

Als Synonym für die biostabile und/oder bioabbaubare Polymerschicht wird im Rahmen der vorliegenden Erfindung der Begriff Beschichtung oder polymere Trägermatrix verwendet.

Eine biostabile und/oder bioabbaubare Polymerschicht im Sinne der Erfindung ist eine zumindest abschnittsweise Auftragung der Komponenten der Beschichtung auf das Medizinprodukt. Vorzugsweise wird die gesamte Oberfläche des Medizinprodukts von der Beschichtung bedeckt. Die Schichtdicke liegt vorzugsweise im Bereich von 0,5 µm bis 30 µm, besonders bevorzugt von 1 µm bis 12 µm.

Der Gewichtsanteil einer erfindungsgemäßen polymeren Trägermatrix an den die Beschichtung bildenden Komponenten der Beschichtung beträgt vorzugsweise mindestens 40%, besonders bevorzugt mindestens 70%. Der Gewichtsanteil des Inhibitors an den die Beschichtung bildenden Komponenten der Beschichtung beträgt bevorzugt nicht mehr als 30%, besonders bevorzugt nicht mehr als 15%.

Die Beschichtung kann direkt auf das Medizinprodukt aufgetragen werden. Die Verarbeitung kann nach Standardverfahren für die Beschichtung erfolgen. Es können einschichtige, aber auch mehrschichtige Systeme (zum Beispiel sogenannte base coat-, drug coat- oder top coat-Schichten) erstellt werden. Die Beschichtung kann unmittelbar auf dem Grundkörper des Implantats aufgebracht sein oder es sind weitere Schichten dazwischen vorgesehen, die beispielsweise der Haftvermittlung dienen.

Alternativ kann die, den Inhibitor enthaltende biostabile und/oder bioabbaubare Polymerschicht als Kavitätenfüllung vorliegen oder Bestandteil einer Kavitätenfüllung sein. Das Medizinprodukt, insbesondere der Stent, weist zu diesem Zweck eine oder mehrere Kavitäten auf. Kavitäten befinden sich beispielsweise an der Oberfläche des Medizinprodukts und lassen sich beispielsweise durch Laserablation in Nano- bis Mikrometerdimensionen erstellen. Bei Medizinprodukten, insbesondere Stents, mit einem biodegradierbaren Grundkörper kann eine Kavität auch im Innern des Grundkörpers angeordnet sein, so dass die Freisetzung des Material erst nach Freilegung erfolgt. Der Fachmann kann sich bei der Ausgestaltung der Kavität an dem im Stand der Technik beschriebenen Systemen orientieren. Der Begriff "Kavität" umfasst dabei z.B. Löcher und Vertiefungen.

Die biostabile und/oder bioabbaubare Polymerschicht im Rahmen der vorliegenden Erfindung ist zusammengesetzt aus Polymeren ausgewählt aus der Gruppe bestehend aus nichtresorbierbaren, permanenten Polymeren und/oder resorbierbaren bioabbaubaren Polymeren.

Besonders bevorzugt ist die biostabile und/oder bioabbaubare Polymerschicht zusammengesetzt aus Polymeren ausgewählt aus der Gruppe Polyolefine, Polyetherketone, Polyether, Polyvinylalkohole, Polyvinylhalogenide, Polyvinylester, Polyacrylate, Polyhalogenolefine, Polyamide, Polyamidimide, Polysulfone, Polyester, Polyurethane, Silikone, Polyphosphazene, Polyphenylen, Polymerschäume (aus Styrolen und Carbonaten), Polydioxanone, Polyglycolide, Polylactide, Poly-ε-caprolacton, Ethylvinylacetat, Polyethylenoxid, Polyphosphorylcholin, Polyhydroxybuttersäuren, Lipide, Polysaccharide, Proteine, Polypeptide sowie Copolymere, Blends und Derivate dieser Verbindungen.

Ganz besonders bevorzugt ist die biostabile und/oder bioabbaubare Polymerschicht zusammengesetzt aus Polymeren ausgewählt aus der Gruppe bestehend aus Polypropylen, Polyethylen, Polyisobutylen, Polybutylen, Polyetheretherketon, Polyethylenglycol, Polypropylenglycol, Polyvinylalkohole, Polyvinylchlorid, Polyvinylfluorid, Polyvinylacetat, Polyethylacrylat, Polymethylacrylat, Polytetrafluorethylen, Polychlortrifluorethylen, PA 11, PA 12, PA 46, PA 66, Polyamidimide, Polyethersulfon, Polyphenylsulfon, Polycarbonate, Polybutylenterephthalat, Polyethylenterephthalat, Elastane, Pellethane, Silikone, Polyphosphazene, Polyphenylen, Polymerschäume (aus Styrolen und Carbonaten), Polydioxanone, Polyglycolide, Poly-L-, Poly-D-, und Poly-D,L-Lactid, sowie Poly-ε-caprolacton, Ethylvinylacetat, Polyethylenoxid, Polyphosphorylcholin, Polyhydroxyvalerat, Cholesterin, Cholesterinester, Alginat, Chitosan, Levan, Hyaluronsäure, Uronide, Heparin, Dextran, Cellulose, Fibrin, Albumin, Polypeptide und Copolymere, Blends und Derivate dieser Verbindungen.

Die biostabile und/oder bioabbaubare Polymerschicht richtet sich bevorzugt nach der gewünschten Elutionsgeschwindigkeit sowie den individuellen Charakteristika der verschiedenen eingesetzten Wirkstoffe und nach der unterschiedlichen Resorptions- bzw. Degradationsgeschwindigkeit am Wirkort des Medizinproduktes.

Der Inhibitor des TRPC-3 Ionenkanals wird bevorzugt in einer Konzentration zwischen 0,25 bis 7 µg/mm² Stentoberfläche, besonders bevorzugt zwischen 0,6 bis 3,8 µg/mm² Stentoberfläche unter, in und/oder auf die biostabile und/oder bioabbaubare Polymerschicht ein- oder aufgebracht.

In einer bevorzugten Ausführungsform liegt die biostabile und/oder bioabbaubare Polymerschicht abluminal auf dem Stent beschichtet vor. Dies hat den Vorteil, dass der in der biostabilen und/oder bioabbaubaren Polymerschicht befindliche Inhibitor nach der Implantation des Stents nur mit dem gereizten Gewebe direkt in Kontakt kommt und somit seine Wirkung lokal ausüben kann und die luminale Oberfläche des Stents frei von jeglichen gegebenenfalls reizauslösenden beschichteten Bestandteilen, wie polymere Trägermatrix oder pharmazeutischer Wirkstoff, ist.

Das erfindungsgemäße Medizinprodukt kann zusätzlich zum Inhibitor noch weitere pharmazeutische Wirkstoffe aufweisen. Dabei kann sich der zusätzliche pharmazeutische Wirkstoff unter, in und/oder auf der biostabilen und/oder bioabbaubaren Polymerschicht befinden. Der pharmazeutische Wirkstoff ist bevorzugt aus den folgenden Arzneistoffklassen ausgewählt: antiangiogene, antimikrobielle, antimitotische, antimyotische, antineoplastische, antiphlogistische, antiproliferative, antithrombotische und vasodilatatorische Mittel.

Insbesondere ist der pharmazeutische Wirkstoff ausgewählt aus der Gruppe umfassend Dexamethason, Methylprednisolon, Diclophenac, Paclitaxel, Colchicin, Actinomycin D, Methotrexat, Limus-Verbindungen, Sirolimus (Rapamycin), Myolimus, Novolimus, Zotarolimus (Abt-578), Tacrolimus (FK-506), Everolimus, Biolimus, insbesondere Biolimus A9, und Pimecrolimus, Deforloimus, Novolimus, Cyclosporin A, Mycophenolsäure, Abciximab, Iloprost, Simvastatin, Mevastatin, Atorvastatin, Lovastatin, Pitavastatin, Pravastatin, Fluvastatin, 17b-Estradiol, Daizein, Genistein, Fibrate, Immunsuppressiva, Sartane, Calciumkanalblocker, Tacrolimus, Imidazole, Antiallergika, Decoy-Oligodesoxynukleotid (dODN), Fibrin, Steroide, Proteinen/Peptiden, Analgetika, Antirheumatika, Bosentan, Fasudil, RGD-Peptide und zyklische RGD (cRGD) (umfassend die Sequenz Arg-Gly-Asp), organische Gold- oder Platinverbindungen, Triclosan, Cephalosporin, Aminoglycosid, Nitrofurantoin, Penicilline, Oxacillin sowie Sulfonamide, Metronidazol, 5-Fluoruracil, Vinblastin, Vincristin, Epothilone, Endostatin, Verapamil, Angiostatin, Angiopeptin, Methotrexat, Colchicin, Flavopiridol, Suramin, Clotrimazol, Flucytosin, Griseofulvin, Ketoconazol, Miconazol, Nystatin, Terbinafin, Prednisolon, Corticosteron, Budesonid, Östrogen, Hydrocortison, Mesalamin, Sulfasalazin, Heparin und seine Derivate, Urokinase, PPack, Argatroban, Aspirin, Abciximab, synthetisches Antithrombin, Bivalirudin, Enoxoparin, Hirudin, r-Hirudin, Protamin, Prourokinase, Streptokinase, Warfarin, 7,3',4'-trimethoxyflavon, Dipyramidol, Trapidil und Nitroprusside.

Die pharmazeutischen Wirkstoffe werden einzeln oder kombiniert in gleicher oder unterschiedlicher Konzentration eingesetzt.

Das erfindungsgemäße Medizinprodukt kann eine weitere, innere oder äußere Beschichtung aufweisen. Eine weitere äußere Schicht kann die Beschichtung oder Kavitätenfüllung enthaltend den Inhibitor ganz oder in Teilen bedecken. Diese äußere Beschichtung kann ein degradierendes Polymer enthalten oder daraus bestehen, insbesondere Polylactide, PLGA (poly(lactic-co-glycolic acid)) oder PLGA-PEG Blockcopolymere. Gegebenenfalls kann in diese weitere, äußere Schicht ein weiterer Wirkstoff eingebettet sein, der frei eluieren kann oder bei Degradation der äußeren Beschichtung freigesetzt wird.

In einer bevorzugten Ausgestaltung der Erfindung wird der Inhibitor direkt auf die Implantatoberfläche aufgebracht und mit einer biostabilen und/oder bioabbaubaren Polymerschicht beschichtet. Gegebenenfalls, falls der Grundkörper des Stents aus einem metallischen Material besteht und eine passivierende Siliziumcarbid-Schicht (SiC) auf dem metallischen Material vorliegt, wird der Inhibitor auf die SiC-Oberfläche aufgebracht und anschließend mit einer biostabilen und/oder bioabbaubaren Polymerschicht beschichtet. Bevorzugte Polymere sind solche, die sich durch Hydrolyse abbauen und deren Abbauprodukte unter anderem eine oder mehrere Carboxylfunktionen enthalten wie Polyester und Polyamide oder biostabile Polymere wie PTFE, Parylen, Silicone oder Urethane. Diese Polymerbeschichtung kann zusätzlich einen weiteren pharmazeutischen Wirkstoff enthalten.

Ein weiterer Aspekt der Erfindung ist die Verwendung von Amidophenylpyrazol oder ein Derivat hiervon, insbesondere Amidophenyl-5-trifluormethylpyrazol oder ein Derivat hiervon, insbesondere bevorzugt Ethyl-1-(4-(2,3,3-trichloracrylamid)phenyl)-5-(trifluormethyl)-1H-pyrazol-4-carboxylat als Inhibitor des TRPC-3 Ionenkanals zur Herstellung eines ganz oder in Teilen mit einer biostabilen und/oder bioabbaubaren Polymerschicht überzogen Medizinproduktes, insbesondere Ballonkatheter oder Stents, wobei sich unter, in und/oder auf der biostabilen und/oder bioabbaubaren Polymerschicht ein Inhibitor des TRPC-3 Ionenkanals, bevorzugt ein Amidophenylpyrazol oder ein Derivat hiervon, insbesondere Amidophenyl-5-trifluormethylpyrazol oder ein Derivat hiervon, insbesondere bevorzugt Ethyl-1-(4-(2,3,3-trichloracrylamid)phenyl)-5-(trifluormethyl)-1H-pyrazol-4-carboxylat befindet.

Ein weiterer Aspekt der Erfindung ist die Verwendung von Amidophenylpyrazol oder ein Derivat hiervon, insbesondere Amidophenyl-5-trifluormethylpyrazol oder ein Derivat hiervon, insbesondere bevorzugt Ethyl-1-(4-(2,3,3-trichloracrylamid)phenyl)-5-(trifluormethyl)-1H-pyrazol-4-carboxylat zur Prophylaxe oder Therapie einer Restenose oder einer Gefäßlumenbeeinträchtigung in einem Gefäßabschnitt.

Ein weiterer Aspekt der Erfindung ist die Verwendung Amidophenylpyrazol oder ein Derivat hiervon, insbesondere Amidophenyl-5-trifluormethylpyrazol oder ein Derivat hiervon, insbesondere bevorzugt Ethyl-1-(4-(2,3,3-trichloracrylamid)phenyl)-5-(trifluormethyl)-1H-pyrazol-4-carboxylat als antiproliferativer Wirkstoff.

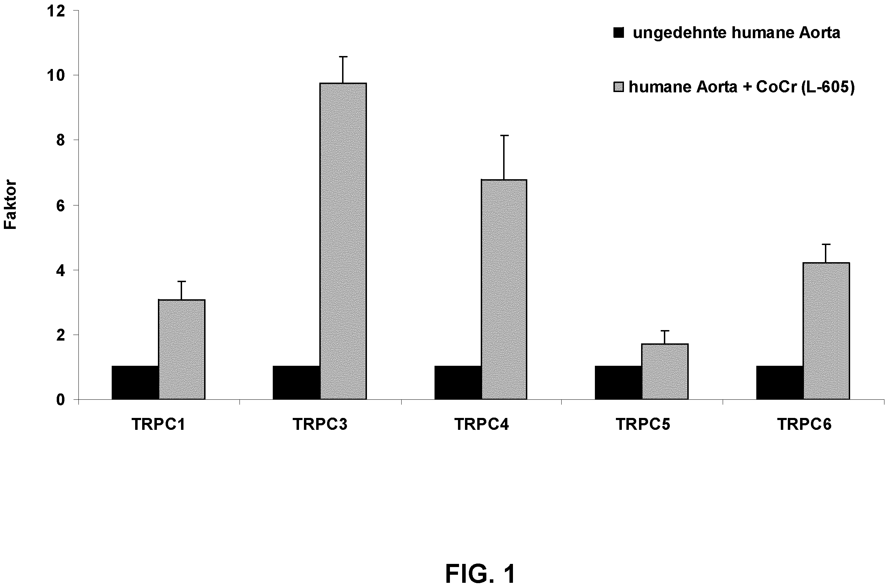

Die Figur 1 zeigt ein Diagramm, welches das Aktivierungsmuster der TRPC Ionenkanälen nach erfolgter mechanischer Gewebeüberdehnung, wie sie normalerweise bei einer Stentimplantation mit einem handelsüblichen Cobaltchromstent auftritt, wiedergibt. Auffällig ist die starke Aktivierung des TRPC-3 Kanals, die bei der Ausbildung einer (Re)Stenose eine wesentliche Rolle spielt.

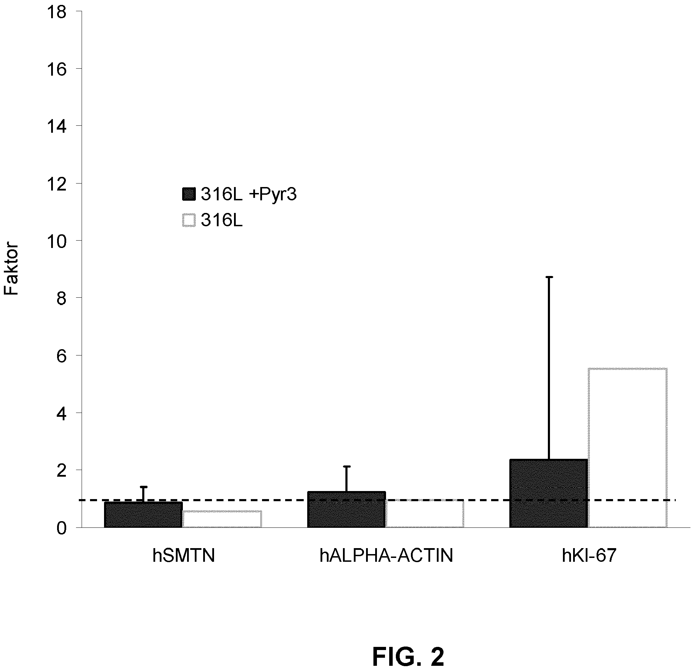

Die Figur 2 zeigt ein Diagramm, welches den Einfluss eines erfindungsgemäßen Stents mit einem TRPC-selektiven Inhibitor (Pyr3) im Vergleich zu einem unbeschichteten Kontrollstent (316L) auf den wichtigen Proliferationsmarker (hKI-67) und die Differenzierung von glatten Muskelzellen (hSMTN) sowie Gesamtzahl α-Actin haltiger Muskelzellen (ALPHA-Actin) wiedergibt.

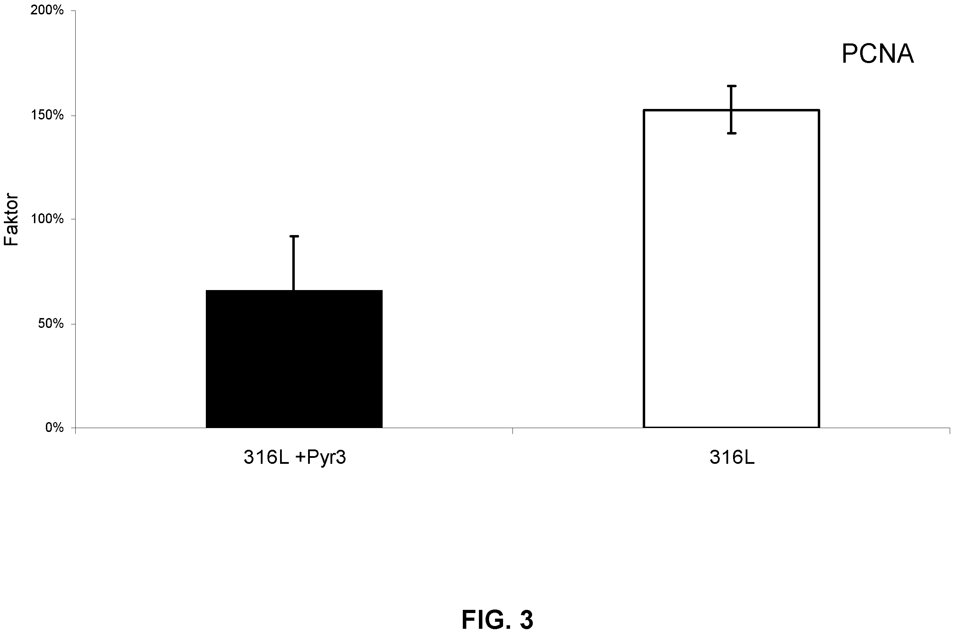

Die Figur 3 zeigt ein Diagramm, welches den Einfluss eines erfindungsgemäßen Stents mit einem TRP-selektiven Inhibitor (Pyr3) im Vergleich zu einem unbeschichteten Kontrollstent (316L) auf die einen weiteren Proliferationsmarker (PCNA) wiedergibt.

### Ausführungsbeispiel 1

Ein Stent aus medizinischem Edelstahl wird wie folgt beschichtet:

Es wird eine Lösung von Ethyl-1-(4-(2,3,3-trichloracrylamid)phenyl)-5-(trifluormethyl)-1H-pyrazol-4-carboxylat (M= 456,63 g/mol) in THF (10 Gew.%) bei Raumtemperatur angesetzt. Diese Lösung wird mit einer zweiten Lösung aus Polylactid (L210; Fa. Boehringer-Ingelheim) in THF (10 Gew%) derart bei Raumtemperatur gemischt, dass Medikament und Polylactid ein Gew.-Verhältnis von 1:1 aufweisen.

Der Stent wird von Staub und Rückständen gereinigt und in eine geeignete Stentbeschichtungsapparatur eingespannt. Mit Hilfe eines Airbrush Systems (Fa. EFD oder Spraying System) wird der sich drehende Stent unter konstanten Umgebungsbedingungen (Raumtemperatur; 42% Luftfeuchtigkeit) halbseitig mit der Goldsalz/Polymermischung beschichtet. Bei einem Düsenabstand von 20 mm ist ein 18 mm langer Stent nach ca. 10 min beschichtet. Nach Erreichen der beabsichtigten Schichtmasse wird der Stent 5 min bei Raumtemperatur getrocknet, ehe, nach Drehen des Stents und erneutem Einspannen, die unbeschichtete Seite auf dieselbe Weise beschichtet wird. Der fertig beschichtete Stent wird für 36 h bei 40°C in einem Vakuumofen (Vakucell; Fa. MMM) getrocknet.

Die Schichtdicke der aufgetragenen Beschichtung beträgt etwa 3-5 µm.

### Ex vivo Test am Aortenmodell

Der ex vivo Test an pathologisch veränderten humanen Arterien und insbesondere an humanen Aorten ist ein neuartiges Testmodell, das es ermöglicht, die klinische Wirksamkeit von biologisch aktiven Beschichtungen von medizinischen Implantaten, beispielsweise Stents oder Ballonkathetern, besser vorhersagen zu können. Diese Vorhersage war bislang nicht zuverlässig möglich. Die Liste derjenigen Substanzen ist lang (z.B. CalciumkanalBlocker, Aspirin, Heparin, Pimecrolimus), die im experimentellen Tiermodell eine gute Wirksamkeit gezeigt haben, überraschenderweise aber in der klinischen Praxis zu keiner Verringerung der Restenose geführt haben. Diese Misserfolge haben am Nutzen der Standardtiermodelle zweifeln lassen, da trotz zusätzlicher Reihenuntersuchungen und großflächiger Screenings die klinische Wirksamkeit einer neuen Substanz trotz positiver präklinischer Ergebnisse ungewiss ist. Bislang gibt es kein Testmodell, welches die antiproliferative Wirkung neuer Medikamente in der komplexen biologischen Umgebung eines arteriosklerotischen Gefäßes untersucht. Anhand dieses neuartigen Testmodells können nun gleichzeitig mechanistische Einblicke in die Entstehung einer Restenose nach Stentimplantation gewonnen und eine ursachenspezifische Therapiemöglichkeit entwickelt werden.

In einem ex vivo Experiment mit pathologisch veränderten humanen Aorten konnte zum ersten Mal gezeigt werden, dass durch eine Stentimplantation und die damit verbundene Gewebsreizung durch die Dehnung des Gefäßes ("Stretch") das Aktivierungsmuster der TRPC Ionenkanäle verändert wird (Figur 1). Bei diesem Experiment wurden in veränderte humane Aorten ex vivo handelsübliche unbeschichtete Stents, bspw. aus einer Cobaltchromlegierung, implantiert und das Gefäß 14 Tage in einem Kulturbad unter Standardbedingungen ausgelagert. Anschließende wurden mittels PCR die Aktivierung der Kanäle TRPC-1 bis TRP-C6 quantitativ bestimmt und mit einem ungestenteten Aortenbereich verglichen, der ebenfalls für 14 Tage im Kulturbad ausgelagert war. Vor allem der TRPC-3 Ionenkanal, der mit Proliferation assoziiert wird, wird dabei im Vergleich zu einem ungestenteten Kontrollabschnitt stark aktiviert.

Es ist nun erstmalig gelungen, ein ex vivo Testmodell zu entwickeln, das die oben genannten Nachteile überwindet. Beim ex vivo Test am Aortenmodell konnten nun zuverlässig ex vivo Testergebnisse in der klinischen Praxis bestätigt werden, wesentlich zuverlässiger als bisher die klinische Wirksamkeit von antiproliferativen Medikamenten und Drug Eluting Stentsystemen vorhergesagt werden konnte.

Das spezielle Testverfahrens basiert auf der Verwendung von humanem pathologischem Gefäßgewebe, welches direkt innerhalb einer Stunde nach Operation (Vasektomie) erhalten wurde. Die Gefäßpräparate werden direkt nach Entnahme in einen sterilen Phosphatpuffer (PBS) bei Raumtemperatur gegeben und transportiert.

In einem 1. Schritt wird bei den humanen, pathologisch veränderten Explantaten, wie beispielsweise Aortensegmenten, die bei einer Bypassoperation entfernt werden, die Adventitia unter aseptischen Bedingungen von umliegenden Muskelgewebe befreit und mit PBS gespült, welches Standard-Antibiotika enthält, und längs aufgeschnitten, wodurch Streifen von 15-20mm Länge erhalten werden. Diese Segmente werden über einen Zylinder mit Standard-chirurgischer Technik (Polyamide/ Nylon) in der Art neu zusammengenäht, dass nach Entfernung des Zylinders Röhren von ca. 3mm Durchmesser erhalten werden. In diese plaquehaltigen, zusammengenähten Explantate wird mittels eines Ballonkatheters ein 3mm/15 Stent mit 14 bar implantiert, wodurch durch die Überdehnung des Gefäßes ein Proliferationsreiz ausgelöst wird. Das gestentete Explantat wird nun für 2 Wochen in einer DMEM Nährlösung ohne Wachstumsfaktoren unter Standardbedingungen (erhöhte Luftfeuchtigkeit, 5% CO2; 37°C) kultiviert. Anschließend wird der Stent entfernt und das Explantat homogenisiert. Nach einigen Aufarbeitungsschritten wird eine PCR durchgeführt.

Sämtliche Proben der Figuren 1 bis 3 wurden nach diesem Testmodell untersucht.

### Ex vivo Test am Aortenmodell von Ausführungsbeispiel 1

Das humane, pathologisch veränderte Explantat wird längs aufgeschnitten und über einem Zylinder auf einen Durchmesser von 2,5 mm neu zusammengenäht. Anschließend wird der erfindungsgemäße Stent nach Ausführungsbeispiel 1 in das zusammengenähte Explantat eingeführt und mit einer Überdehnung implantiert. Anschließend wird der Test wie oben beschrieben durchgeführt.

### Als Vergleichsprobe dient der nicht erfindungsgemäße unbeschichtete Stent aus 316L.

Figur 2 zeigt den Einfluss des erfindungsgemäßen Stents nach Ausführungsbeispiel 1 auf die Proliferation und die Differenzierung von glatten Muskelzellen. Dabei ist die gestrichelte Linie in Figur 2 der Normwert, der für ein ungedehntes Gefäß bei 1 liegt.

Dabei wurde an 3 unterschiedlichen Zellkulturen getestet: hSMTN, hALPHA-Actin und hKI-67. Hierbei handelt es sich um:
hSMTN: Smoothelin; Marker für Muskelzellen des kontraktilen Phenotyps
hALPHA-Actin: Marker für alle Arten von glatten Muskelzellen
hKI-67: Proliferationsmarker monoklonaler Antikörper KI 67

Figur 3 gibt die Proliferationsaktivität des erfindungsgemäßen Stents auf den Proliferationsmarker PCNA wieder.

In Figur 1 ist gut zu erkennen, wie die mechanische Dehnung der pathologisch veränderten Aorta das Aktivierungsmuster der verschiedenen TRPC-Kanäle verändert. Die Aktivierung wird durch das Maß an Gefäßverletzung bestimmt, welche von der Art her einer normalen Gefäßdilatation bei Behandlung einer Stenose vergleichbar ist. Nach bisherigen Erkenntnissen werden vor allem die mechanosensitiven Kanäle TRPC-1 und TRPC-3 mit der Proliferation assoziiert, wobei TRPC-3 am stärksten auf den mechanischen Stimulus reagiert und den entsprechenden Ionenkanal um fast Faktor 10 im Vergleich zu einem ungedehnten Gefäßabschnitt hoch reguliert.

In Figur 2 und 3 ist zuerkennen, wie der TRPC-3-selektive Inhibitor die Proliferation (gemessen an den beiden wichtigen Proliferationsmarkern KI-67 und PCNA) beeinflusst, wenn er aus einer polymeren Stentbeschichtung abgegeben wird. Dabei wird jeweils ein herkömmlicher Stent ohne Medikament (316L) verglichen mit dem gleichen Stent, der einen selektiven TRPC-3 Inhibitor (Pyr3) gemäß Ausführungsbeispiel 1 aufweist. Anhand der Exprimierung von hSMTN und hAlpha-Actin kann man in Figur 2 erkennen, dass eine Inhibierung des TRPC-3 Ionenkanal mit Pyr3 nicht zu dem sonst üblichen Abfall der Gesamtzahl an glatten Muskelzellen nach Stentimplantation führt, wodurch offensichtlich auch der biologische Reiz geringer ist, diese zu ersetzen, was sich in einer geringeren Gesamtproliferation äußert.

Durch die lokale Anwendung selektiver Inhibitoren mit einer hohen Affinität gegenüber den zellulären TRPC Ionenkanäle konnte gezeigt werden, dass die stretch-induzierte Proliferation in einer pathologisch veränderten, humanen Aorta nach Stentimplantation deutlich verringert werden konnte. Dieses Ergebnis ist überraschend, da andere Calciumkanalblocker, die nicht selektiv auf einen der TRP-Kanäle wirken, im klinischen Einsatz keine antirestenotische Wirkung aufweisen.

## Patentansprüche

1. Medizinprodukt, wobei die Oberfläche des Medizinprodukts ganz oder in Teilen mit einer biostabilen und/oder bioabbaubaren Polymerschicht überzogen ist und sich unter, in und/oder auf der biostabilen und/oder bioabbaubaren Polymerschicht ein Inhibitor des TRPC-3 Ionenkanals befindet.

2. Medizinprodukt nach Anspruch 1, **dadurch gekennzeichnet, dass** der Inhibitor des TRPC-3 Ionenkanals ein Amidophenylpyrazol oder ein Derivat hiervon ist.

3. Medizinprodukt nach Anspruch 2, **dadurch gekennzeichnet, dass** der Inhibitor des TRPC-3 Ionenkanals Ethyl-1-(4-(2,3,3-trichloracrylamid)phenyl)-5-(trifluormethyl)-1H-pyrazol-4-carboxylat ist.

4. Medizinprodukt nach einem der vorhergehenden Ansprüche, **dadurch gekennzeichnet, dass** die biostabile und/oder bioabbaubare Polymerschicht zusammengesetzt ist aus Polymeren ausgewählt aus der Gruppe bestehend aus Polyolefine wie Polypropylen, Polyethylen, Polyisobutylen und Polybutylen, sowie Polyetherketone wie Polyetheretherketon, sowie Polyether wie Polyethylenglycol und Polypropylenglycol, sowie Polyvinylalkohole, Polyvinylhalogenide wie Polyvinylchlorid und Polyvinylfluorid, sowie Polyvinylester wie Polyvinylacetat, sowie Polyacrylate wie Polyethylacrylat, Polymethylacrylat und Polymethylmetacrylat, sowie Polyhalogenolefine wie Polytetrafluorethylen und Polychlortrifluorethylen, sowie Polyamide wie PA 11, PA 12, PA 46 und PA 66, sowie Polyamidimide, Polysulfone wie Polyethersulfon und Polyphenylsulfon, sowie Polyester wie Polycarbonate, Polybutylenterephthalat, Polyethylenterephthalat, sowie Polyurethane wie Elastane und Pellethane, sowie Silikone, Polyphosphazene, Polyphenylen, Polymerschäume (aus Styrolen und Carbonaten), Polydioxanone, Polyglycolide, Polylactide wie Poly-L-, Poly-D-, und Poly-D,L-Lactid, sowie Poly-ε-caprolacton, Ethylvinylacetat, Polyethylenoxid, Polyphosphorylcholin, Polyhydroxybuttersäuren wie Polyhydroxyvalerat, sowie Lipide wie Cholesterin und Cholesterinester, sowie Polysaccharide wie Alginat, Chitosan, Levan, Hyaluronsäure, Uronide, Heparin, Dextran und Cellulose, sowie Proteine wie Fibrin und Albumin, sowie Polypeptide und Copolymere, Blends und Derivate dieser Verbindungen.

5. Medizinprodukt nach einem der vorhergehenden Ansprüche, **dadurch gekennzeichnet, dass** sich unter, in und/oder auf der biostabilen und/oder bioabbaubaren Polymerschicht der Inhibitor des TRPC-3-Kanals in einer Konzentration von 0,25 bis 7 µg/mm² befindet.

6. Medizinprodukt nach ein der vorhergehenden Ansprüche, **dadurch gekennzeichnet, dass** sich unter, in und/oder auf der biostabilen und/oder bioabbaubaren Polymerschicht zusätzlich ein pharmazeutischer Wirkstoff befindet.

7. Medizinprodukt nach einem der vorhergehenden Ansprüche, **dadurch gekennzeichnet, dass** das Medizinprodukt ein Ballonkatheter oder ein Stent ist.

8. Medizinprodukt nach Anspruch 7, **dadurch gekennzeichnet, dass** der Stent aus einem metallischen Material aus einem oder mehreren Metallen aus der Gruppe Eisen, Magnesium, Nickel, Wolfram, Titan, Zirkonium, Niob, Tantal, Zink, Platin, Iridium oder Silizium besteht.

9. Medizinprodukt nach Anspruch 7, **dadurch gekennzeichnet, dass** die biostabile und/oder bioabbaubare Polymerschicht abluminal auf dem Stent beschichtet vorliegt.

10. Verwendung von Amidophenylpyrazol oder ein Derivat hiervon als Inhibitor des TRPC-3 Ionenkanals zur Herstellung eines Medizinproduktes nach einem der Ansprüche 1 bis 9.

11. Verwendung Nach Anspruch 10, wobei der Inhibitor Ethyl-1-(4-(2,3,3-trichloracrylamid)phenyl)-5-(trifluormethyl)-1H-pyrazol-4-carboxylat ist.

12. Verwendung von Amidophenylpyrazol oder ein Derivat hiervon zur Prophylaxe oder Therapie einer Restenose oder einer Gefäßlumenbeeinträchtigung in einem Gefäßabschnitt.

13. Verwendung nach Anspruch 12, wobei das Derivat Ethyl-1-(4-(2,3,3-trichloracrylamid)phenyl)-5-(trifluormethyl)-1H-pyrazol-4-carboxylat ist.

14. Verwendung von Amidophenylpyrazol oder ein Derivat hiervon als antiproliferativer Wirkstoff.

15. Verwendung nach Anspruch 14, wobei das Derivat Ethyl-1-(4-(2,3,3-trichloracrylamid)phenyl)-5-(trifluormethyl)-1H-pyrazol-4-carboxylat ist.
